(19) Europäisches Patentamt
European Patent Office
Office européen des brevets

(11) EP 2 408 440 B1

(12) **EUROPEAN PATENT SPECIFICATION**

(45) Date of publication and mention of the grant of the patent:
**11.05.2016 Bulletin 2016/19**

(21) Application number: **10753026.3**

(22) Date of filing: **18.03.2010**

(51) Int Cl.:
***A61K 38/12*** *(2006.01)* ***A61K 31/45*** *(2006.01)*
***A61K 9/70*** *(2006.01)* ***A61M 37/00*** *(2006.01)*
***A61P 7/02*** *(2006.01)* ***A61K 31/4465*** *(2006.01)*
***A61F 13/02*** *(2006.01)* ***A61K 31/4515*** *(2006.01)*

(86) International application number:
**PCT/CA2010/000373**

(87) International publication number:
**WO 2010/105342 (23.09.2010 Gazette 2010/38)**

(54) **TRANSDERMAL PHARMACEUTICAL PREPARATION AND ADMINISTRATION OF TIROFIBAN**

TRANSDERMALE PHARMAZEUTISCHE ZUBEREITUNG UND VERABREICHUNG VON TIROFIBAN

PRÉPARATION PHARMACEUTIQUE TRANSDERMIQUE ET ADMINISTRATION DE TIROFIBAN

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **18.03.2009 US 161132 P**
**04.09.2009 US 240021 P**
**10.11.2009 US 259683 P**

(43) Date of publication of application:
**25.01.2012 Bulletin 2012/04**

(73) Proprietor: **Medicure International Inc.**
**Holetown 24116**
**Saint James (BB)**

(72) Inventors:
- **THOMAS, George Roby**
  **Winnipeg**
  **Manitoba R3P 2R3 (CA)**
- **REIMER, Dawson James**
  **Winnipeg**
  **Manitoba R3Y 1X1 (CA)**
- **FRIESEN, Albert D.**
  **Winnipeg**
  **Manitoba R3P 1P4 (CA)**

(74) Representative: **Nederlandsch Octrooibureau**
**P.O. Box 29720**
**2502 LS The Hague (NL)**

(56) References cited:
**WO-A1-2004/036226** **WO-A2-02/03969**
**CA-A1- 2 483 929** **US-A1- 2003 207 918**

- **COOK ET AL.: 'Tirofiban (Aggrastat(TM)' CARDIOVASCULAR DRUG REVIEWS vol. 17, no. 3, September 1999, pages 199 - 224, XP055092027**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### Cross Reference to Related Application

This application claims the benefit of and priority to United States Patent Application Nos. 61/161,132 filed March 18, 2009, under the title A METHOD FOR INHIBITING PLATELET AGGREGATION, 61/240,021, filed September 4, 2009, under the title TRANSDERMAL PHARMACEUTICAL PREPARATION AND ADMINISTRATION OF TIROFIBAN, and 61/259,683, filed November 10, 2009 under the title TRANSDERMAL PHARMACEUTICAL PREPARATION AND ADMINISTRATION OF TIROFIBAN.

The content of the above patent applications is hereby expressly incorporated by reference into the detailed description hereof.

### Field of the Invention

This invention relates to transdermal delivery of tirofiban.

### Background of the Invention

Inhibitors of the glycoprotein complex GP IIb/IIIa include abciximab, tirofiban, and eptifibatide, and are currently used intravenously to inhibit platelet aggregation acutely in a hospital setting. Inhibition of platelet aggregation results in reduced incidences or reduced severity of adverse events such as damage to the heart. Typical use of these inhibitors involves initial larger loading bolus injection and subsequent slower sustained infusion, for a period of hours or days to maintain adequate platelet inhibition

WO02/03969 discloses transdermal delivery systems (adhesive layer, backing, pharmaceutical composition) with skin penetration enhancers, such oleic acid in the examples.

US 2003/207918 and WO 2004/036226 disclose the inhibition of platelets by tirofiban, its use in related indications (thrombosis, infarction, stroke etc.) and the IV administration.

Tirofiban hydrochloride monohydrate (Aggrastat®), chemically described as N-butylsulfonyl-O-[4-(4-piperidinyl)butyl]-L-tyrosine monohydrochloride monohydrate, is a representative non-peptide reversible antagonist of the platelet glycoprotein (GP) IIb/IIIa receptor. It is a nonpeptide mimetic derived from snake venom. Because GP IIb/IIIa inhibitors block the final common pathway to platelet aggregation, the binding of fibrinogen to GP IIb/IIIa receptors, these agents can provide potent (>80%) inhibition of platelet aggregation, disaggregating existing thrombus and preventing new platelet aggregates from forming.

Tirofiban is approved for the treatment of acute coronary syndrome, including patients to be managed medically or to undergo atherectomy or percutaneous transluminal coronary angioplasty (PTCA). Administration of tirofiban can reduce a combined endpoint of death, new myocardial infarction, refractory ischemia, or repeat cardiac procedure.

Tirofiban is currently administered via intravenous administration at an initial rate of 0.4 mcg/kg/min for 30 minutes and then continued at 0.1 mcg/kg/min or 0.15 mcg/kg/min. A high dose bolus of 15 to 25 mcg/kg for 3-5 minutes can also be administered as an initial dose, with or without an ensuing maintenance infusion depending on the patient and the treatment modality chosen by the physician. It is available as a pre-mixed bag or a vial, both of which are prepared for IV administration. The biological half life of tirofiban is approximately 2 hours, and the level of platelet inhibition is directly related to the drug level in the blood. Therefore when drug infusion is stopped the antiplatelet action stops after a couple hours. To maintain continuous administration of tirofiban to a patient, the premixed IV bags have to be changed routinely throughout the day by the hospital staff. Also, the patient would have to remain in the hospital under close supervision as long as he/she is prescribed tirofiban since IV administration is not approved outside of the hospital setting and its administration requires oversight by healthcare professionals. Therefore, it is not used on a sub-chronic or chronic, outpatient basis.

Common oral antiplatelet drugs, such as acetylsalicylic acid and clopidogrel, are primarily used chronically for purposes such as the prevention of heart attacks, whereas, Gp IIb/IIIa inhibitors are used in acute settings such as following a heart attack and during percutaneous coronary interventions (PCI). Although several attempts were made at developing oral Gp IIb/IIIa inhibitors for chronic use, they have been unsuccessful as many concerns have been uncovered during the clinical trials. Problems include high incidences of minor bleeding events at the dose necessary for platelet inhibition; inter-patient variability with drug levels, pharmacokinetics and platelet inhibition as compared to the IV doses; and limited efficacy (Cannon, 2003).

Tirofiban or other GpIIb/IIIa inhibitors formulated for sub-chronic or chronic use in a delivery system such as a transdermal patch would be a major improvement over the current oral antiplatelet drugs. As an example, clopidogrel is an irreversible antiplatelet agent and therefore takes several days after stopping treatment before it fully loses its effect. If a patient on clopidogrel experiences any sort of nuisance bleeding or more severe bleeding, he would have to

stop treatment while the bleeding risk would continue for a considerable period. The fact that he has stopped treatment also places him at a much higher risk of a blood clot causing a serious health problem. Administering a GpIIb/IIIa in a titratable transdermal patch would allow the patient to more rapidly alleviate the effects of the drug to cease the bleeding and then more quicly return to appropriate platelet inhibition by reapplying a patch. The platelet inhibition could be restored to pre-bleeding levels once the bleeding was stopped.

Transdermal patches, in general, are known, including matrix-type patches, multi-laminate drug-in-adhesive type patches, and monolithic drug-in-adhesive type patches.

All of these patch types are generally fixed dose patches. In a fixed dose patch, the rate of delivery of the drug from the patch to the skin or mucosa of a host, known as the flux rate, is constant and predetermined by the individual patch that is prescribed.

As such, presently, a pharmacist needs to stock multiple patches each containing various dosages of therapeutic agents. For example, where various dosage strengths are indicated or otherwise required, a pharmacist needs to stock separate and different transdermal patches, each having one of the various dosage strengths - for example, different patches need to be stocked for each of dosage strengths such as 1, 2, 4, 10, 20 units per time (milligrams/hour). When a physician prescribes certain dosage strength to a patient, the patient purchases transdermal patches having the fixed dosage of therapeutic agent. If the prescribed amount is too strong (for example, a 20 mg/hr patch supply is originally prescribed and purchased), the patient will typically have to purchase another supply of transdermal patches having a reduced dosage of therapeutic agent. If the prescribed amount is too weak (for example, 2 mg/hr patch supply is originally purchased, and the dosage requirement changes to 5 mg/hr), the patient will typically have to purchase another supply of transdermal patches having an increased dosage of therapeutic agent.

## Summary of the Invention

The present invention relates to a transdermal drug delivery system in the form of a transdermal patch. A transdermal patch of the present invention can be adapted to deliver tirofiban to a patient in a titratable manner. The present invention could also be used as a part of kit to maintain a desired level of platelet inhibition. A transdermal patch of the present invention can be used in treating disorders not currently feasible with the intravenous formulation or in significantly improving the ability to treat disorders for which antiplatelet medications are currently prescribed.

One aspect of the present invention provides a novel adhesive coated sheet material comprising (1) a flexible backing and (2) a pressure sensitive adhesive coating comprising a homogeneous mixture of (a) an acrylic adhesive polymer comprising, a hydrophobic monomeric acrylic or methacrylic acid ester of an alkyl alcohol, the alkyl alcohol containing about 2-10 carbons and (b) tirofiban in an amount by weight of about 1-50% of the total weight of the adhesive coating.

In another aspect of the present invention, transdermal administration of tirofiban, can inhibit platelet aggregation by at least 40% and no more than 60%, at least 50% and no more than 70%, at least 65% and no more than 80%, at least 75% and no more than 90%, at least 85%, or at least 90%. This administration can be achieved by a titratable dosage transdermal delivery system.

In a further aspect of the present invention, an adhesive coated sheet material is suitable for continuous transdermal delivery of tirofiban, to a subject over a prolonged period in an amount that is therapeutically effective for treating angina and other cardiovascular disorders in addition to other disorders.

In a further aspect of the present invention, cardiovascular disorders include a variety of acute, sub-chronic and chronic conditions including acute coronary syndrome, unstable angina, ST-elevated myocardial infarction, non-ST elevated myocardial infarction, ischemic stroke, post CABG with incomplete revascularization, essential thrombocytosis, deep vein thrombosis, pulmonary embolism, patients allergic and /or with ASA resistance, heparin induced thrombocytopenia, and prior to and during/peri-procedural PCI.

In a further aspect of the present invention, other indications include: central and branch vein occlusion and knee and hip replacement surgery.

In another aspect of the invention, a laminated composite is provided that includes a strippable protective release liner laminated to a basal surface of a drug reservoir.

An adhesive coating of the tapes of the invention may optionally comprise a skin penetration enhancer.

In a further aspect of the present invention, a transdermal patch can be used for a prolonged period of treatment by replacing patches at regular basis intervals of time and/or modifying characteristics of the transdermal patch to affect duration of treatment provided by each individual patch.

In another aspect of the present invention a prolonged period of treatment can be 2 hours to one or more years.

In a further aspect of the present invention the prolonged period of treatment for acute or emergent uses can be 2 hours to 3 days.

In yet a further aspect of the present invention the prolonged period of treatment for sub-chronic uses can be 2 days to 30 days.

In another aspect of the present invention the prolonged period of treatment for sub-chronic or chronic uses can be 30 days to one or more years.

In the present invention, tirofiban or a tirofiban salt is used. In addition, other GP IIb/IIIa inhibitors include eptifibatide, abciximab, lamifiban, xemilofiban, sibrafiban, fradafiban, roxifiban, lotrafiban and orbofiban; ADP receptor inhibitors such as ticlopidine, clopidogrel, ticagrelor, and prasugrel; PDE inhibitors such as dipyridamole, and cilostazol; direct thrombin inhibitors such as Ximelagatran, Dabigatran, Argatroban, and bivalirudin; heparin, low molecular weight heparins, novel Factor Xa inhibitors, TF/FVIIa inhibitors and other anticoagulants, antiplatelets and thrombolytics could be delivered transdermally using appropriate formulation strategies. Higher molecular weight agents are less preferable as compared to tirofiban due to formulation, pharmacokinetics and patient compliance issues.

In a further aspect, the invention includes a transdermal drug delivery system comprising a sheet material coated with an adhesive on a first side; a pharmaceutical composition contacting a second side of said sheet material and capable of at least partially passively diffusing through said sheet material to said first side; and a flexible backing; wherein the flexible backing and the adhesive-coated sheet material form a pocket containing said pharmaceutical composition; the pharmaceutical composition is incapable of passively diffusing through the flexible backing; the pharmaceutical composition comprises tirofiban, or a salt or hydrate thereof; and an adhesive on said adhesive-coated sheet material is capable of adhering to a patient's skin.

In yet a further aspect, a transdermal drug delivery system further comprises a skin permeation or skin penetration enhancer, such as but not limited to microneedle technology and iontophoresis. In a further aspect, a skin permeation device or skin penetration enhancer can be used prior to application of the transdermal patch to the skin. In a further aspect, a skin permeation or skin penetration enhancer is included in the trandermal delivery system or kit.

In a further aspect, a skin permeation device or skin penetration enhancer is coated or impregnated with the active pharmaceutical ingredient in a manner that further speeds or enhances the delivery of the intended dosage to the patient.

In a further aspect, a skin permeation or skin penetration enhancer is located on or within the adhesive - coated sheet material.

In a further aspect, a skin permeation or skin penetration enhancer is selected from the group consisting ofN-methyl-2-pyrrolidone, oleic acid, C8-C22 aliphatic alcohol, sorbitan ester, linoleic acid, and isopropyl linoleate.

In yet a further aspect, a transdermal drug delivery system further comprises a carrier material within said pocket. In yet a further aspect, a carrier material is selected from the group consisting of a liquid, a gel, a solvent, a liquid diluent, and a solubilizer. In yet a further aspect, a carrier material is selected from the group consisting of water, a mineral oil, a silicone, an inorganic gel, an aqueous emulsion, a liquid sugar, a wax, a petroleum jelly, an oil, and a polymeric material.

A further aspect of the invention also includes an adhesive coated sheet material comprising (1) a flexible backing and (2) a pressure sensitive adhesive coating comprising a homogenous mixture of (a) an acrylic adhesive polymer and (b) tirofiban in an amount by weight of about 1-50% of the total weight of the adhesive coating. In yet a further aspect, an acrylic adhesive polymer comprises a hydrophobic monomeric acrylic and/or methacrylic acid ester of an alkyl alcohol, said alkyl alcohol containing about 2 to 10 carbon atoms.

Another embodiment of the present invention is a transdermal patch comprising a) one or more backing layers, b) a matrix layer, wherein the matrix layer comprises a polymeric matrix material, and c) tirofiban or a salt or hydrate thereof in solution or suspension within said polymeric matrix material.

In yet a further aspect, a polymeric matrix material is selected from the group consisting of a polyvinyl alcohol, a polyvinyl pyrrolidone, a gelatin, and combinations thereof.

In yet a further aspect, a pharmaceutical composition comprises eptifibatide, or a salt or hydrate thereof.

In yet a further aspect, a transdermal drug delivery system delivers tirofiban, or a salt or hydrate thereof, at a rate equivalent to approximately 0.10 ug/kg/min.

In yet a further aspect, a transdermal drug delivery system delivers tirofiban, or a salt or hydrate thereof, at a rate equivalent to approximately 0.15 ug/kg/min.

In yet a further aspect, a transdermal drug delivery system delivers tirofiban, or a salt or hydrate thereof, at a rate equivalent to approximately 25 ug/kg in total over a period of 3 - 60 minutes.

In yet a further aspect, a transdermal drug delivery system comprises tirofiban and is capable of adhering to a patient and, when adhered to a patient, is capable of delivering tirofiban to said patient.

An object of the present invention comprises a titratable dosage transdermal delivery system.

In a further aspect of the present invention, a titratable dosage transdermal delivery system includes any one or more of (1) a matrix type patch; (2) a reservoir type patch; (3) a monolithic drug-in-adhesive type patch; and (4) a multi-laminate drug-in-adhesive type patch.

In a further aspect of the present invention a titratable dosage transdermal delivery system comprises tirofiban or a salt or hydrate thereof.

In a further aspect of the present invention, a titratable dosage transdermal delivery system can deliver tirofiban,

to a subject over a prolonged period in an amount which is therapeutically effective and suitably safe for treating the patients specific disorder.

In a further aspect of the present invention, cardiovascular disorders include a variety of acute, sub-chronic and chronic conditions including acute coronary syndrome, unstable angina, ST-elevated myocardial infarction, non-ST elevated myocardial infarction, ischemic stroke, post CABG with incomplete revascularization, essential thrombocytosis, deep vein thrombosis, pulmonary embolism, patients allergic and /or with ASA resistance, heparin induced thrombocytopenia, and prior to and during/peri-procedural PCI.

In another embodiment the invention provides kits for determining the specific degree of platelet inhibition for an individual patient and for titrating the dose level of the antithrombotic agent to ensure the desired effective inhibition level for the individual. The transdermal patch employing an antithrombotic agent like tirofiban can be used in conjunction with a platelet function analysis system, for example, ICHOR/Plateletworks, Ultegra / RPFA system, Accumetrix VerifyNow system. A receptor occupancy assay, or another form of platelet reactivity assay, as known in the art, can also be used. Titration of the inhibition of platelet aggregation can be achieved by increasing the dose of the antithrombotic in a graded stepwise manner.

In a further embodiment of the invention, the kits can include agents for reducing background interference in a test, agents for increasing signal, apparatus for conducting a test, calibration curves and charts, standardization curves and charts and the like.

In a further aspect of the present invention a titratable dosage transdermal delivery system delivers tirofiban, or a salt or hydrate thereof, to the circulatory system at a rate equivalent to approximately 0.1 mg/h to 75 mg/h.

In a further aspect, the titratable dosage transdermal delivery system comprises a patch with one or more divisible borders which indicate the separation of doses. For example, a continuous delivery patch with a total tirofiban dosage of 1 mg/hr can be divided into 10 separate units each capable of delivering 0.1 mg/hr. Thus a patient can initially start at 0.1 mg/hr and increase the dose depending on the level of platelet inhibition needed. As another example, a bolus delivery patch would necessarily need to provide a much higher rate of delivery, delivering a total dose of 1mg to 3 mg over a period of 3 - 60 minutes.

$$6 \text{ ug/kg/hr} \times 100 \text{ kg} \times 24 \text{ hrs} = 14.4 \text{ mg /day}$$

$$10 \text{ ug/kg/hr} \times 100 \text{ kg} \times 24 \text{ hrs} = 24 \text{ mg/day}$$

$$25 \text{ ug/kg} \times 100 \text{ kg} = 2.5 \text{ mg bolus}$$

In a further aspect, the titratable dosage transdermal delivery system includes a series of transdermal patches with varying rates of drug release based on the size and/or characteristics of each patch. For example, a patient can initially start at 0.2 mg/hr and if that dose does not provide the level of platelet inhibition needed, the dose can be increased by removing the patch and applying a patch delivering 0.4 mg/hr. This can be repeated until the desired level of platelet inhibition is achieved.

The titratable dosage transdermal delivery system can also provide a chart or tool that can provide the individual necessary information to accurately adjust and progressively titrate the dosing depending on the specific age, sex, weight, disease state and other specific characteristics of the patient.

A bolus dose, followed by a maintenance dose regime, as described in US patent 6,770,660 (which is incorporated herein by reference) can also be used. In the case of such dosing methodology, either the bolus dose, the maintenance dosing, or both the bolus and maintenance doses can be administered through a transdermal patch as herein described. For example, a set bolus dose can be administered intravenously by a physician, and followed by a variable, patient administered and titrated, transdermally administered, maintenance dosage regimen, as discussed further below.

Detailed **Description**

Transdermal patch administration of tirofiban, would allow maintenance of a stable, predictable drug level and thus maintain an intended target level of platelet inhibition without using an IV continuous infusion. It would provide easier administration, improved safety, better compliance, improved mobility for the patient, reduced hospital resource utilization, and avoidance of the digestive tract (vs. an oral route). It would also facilitate use of these agents in settings for which this class of drug is not currently feasible. It would provide easier, more rapid titration of platelet inhibition to

ensure the optimal balance of efficacy and safety for the individual patient.

Furthermore, instead of fixed dose transdermal patches, a transdermal patch that is titratable (i.e. where a patient or doctor could decide on amount of drug to deliver) would provide a further improvement by allowing a physician or patient to achieve platelet inhibition levels lower than 85% when desired (instead of the typical dosage for tirofiban, which provides a desired >90% inhibition). This would allow tirofiban to be used for chronic indications outside of the hospital where moderate platelet inhibition is necessary, for example, post or pre surgery. It would also allow for changes in dosage administered to the patient without the need for a new supply of patches.

Tirofiban hydrochloride, commercially available as AGGRASTAT®, is a non-peptide antagonist for the glycoprotein IIb/IIIa fibrinogen receptor. Tirofiban hydrochloride is chemically described as *N*-(butylsulfonyl)-*O*-[4-(4-piperidinyl) butyl]-L-tyrosine monohydrochloride and structurally represented as

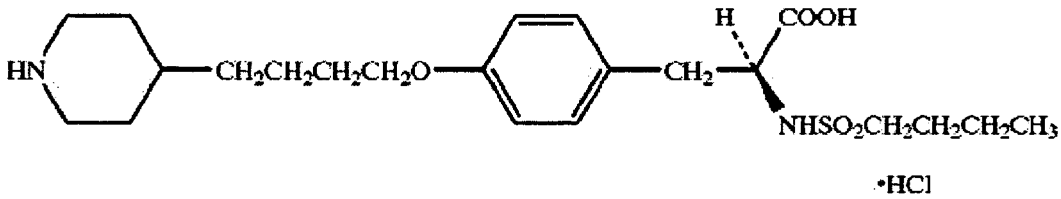

Tirofiban hydrochloride is also referred to as (2-S-(n-Butylsulfonylamino)-3[4-(piperidin-4-yl)butyloxyphenyl] propionic acid hydrochloride, and is described in U.S. Patent No. 5,292,756.

Tirofiban hydrochloride and related pharmaceutically acceptable salts are useful in the present invention. The term "pharmaceutically acceptable salts" means non-toxic salts of the compounds which include, but are not limited to, acetate, benzenesulfonate, benzoate, bicarbonate, bisulfate, bitartrate, borate, bromide, calcium edentate, camsylate, carbonate, chloride, clavulanate, citrate, dihydrochloride, edetate, edisylate, estolate, esylate, fumarate, gluceptate, gluconate, glutamate, glycollylarsanilate, hexylresorcinate, hydrabamine, hydrobromide, hydrochloride, hydroxynapthoate, iodide, isothionate, lactate, lactobionate, laurate, malate, maleate, mandelate, mesylate, methylbromide, methylnitrate, methylsulfate, mucate, napsylate, nitrate, oleate, oxalate, pamaote, palmitate, panthothenate, phosphate/diphosphate, polygalacturonate, salicylate, stearate, subacetate, succinate, tannate, tartrate, teoclate, tosylate, triethiodide, and valerate.

Tirofiban, tirofiban hydrochloride, and other tirofiban salts, are also collectively referred to hereinafter as "active drug."

A release liner is a disposable element designed to protect an exposed reservoir surface prior to use. A release liner, for ease of removal, is preferably a two-part structure in which a first strippable protective sheet partially overlaps a second strippable protective sheet, giving rise to a tab extending from a basal surface of the patch.

Pharmaceutically effective amounts of the active drug are suitable for use in the methods of the present invention. The term "pharmaceutically effective amount" means that amount of a drug or pharmaceutical agent that will elicit the biological or medical response of a tissue, system or animal that is being sought by a researcher or clinician.

The methods employing the transdermal delivery systems of the present invention are useful in combination with other procedures for treating candidate patients, including procedures involving treatments with other anticoagulants (e.g. heparin and warfarin), thrombolytic agents (e.g. streptokinase and tissue plasminogen activator), and platelet antiaggregation agents (e.g. aspirin and dipyridamole) and also may include concurrent use of a medical device (e.g. stent) or medical procedure (e.g. bypass surgery or angioplasty).

A dosage regimen utilizing the active drug is selected in accordance with weight of the patient and in accordance the degree of platelet inhibition clinically required to best treat the specific condition in the individual patient.

The active drug can be administered in admixture with suitable pharmaceutical diluents, excipients or carriers (collectively referred to herein as "carrier" materials) suitably selected with respect to the intended form of administration and consistent with convention pharmaceutical practices.

The methods employing the transdermal delivery systems according to the present invention for administering the active drug are useful for treating patients where inhibition of human or mammalian platelet aggregation or adhesion is desired. They are useful in surgery on peripheral arteries eg. (arterial grafts) and in cardiovascular surgery where manipulation of arteries and organs, and/or the interaction of platelets with artificial surfaces, leads to platelet aggregation and potential formation of thrombi and thromboemboli. A transdermal drug delivery system of the invention may be used to prevent formation of thrombi and thromboemboli. Other applications include prevention of platelet thrombosis, thromboembolism and reocclusion during and after thrombolytic therapy, percutaneous coronary intervention or revascularization and prevention of platelet thrombosis, thromboembolism and reocclusion after angioplasty or coronary artery bypass procedures. The transdermal drug delivery systems may also be used to prevent myocardial infarction.

Transdermal Administration

In an embodiment, tirofiban or a salt thereof can be administered via a transdermal patch. 'Transdermal" refers to passage of a drug through skin and into the bloodstream to achieve effective therapeutic blood levels of the drug. Thereby, a patch comes into contact with the skin or mucosal tissue of a patient and has the ability to deliver a therapeutic level of tirofiban or salts thereof.

A transdermal patch has several advantages compared to oral administration. Oral agents are typically subject to varied bioavailability and pharmacokinetics. As a result it is difficult to predict the drug level in a given patient and thereby to provide the appropriate degree of platelet inhibition. It has also been associated with negative outcomes, including but not limited to excessive bleeding, lack of efficacy, and unwanted side effects, such as gastric complications associated with oral administration. Because a transdermal patch can deliver the antithrombotic agent more predictably and rapidly than by oral delivery, this method of delivery is more suitable for titrating the dose of agent to meet the desired level of platelet inhibition for each specific individual. Because the transdermal patch can also have delivery of the antithrombotic agent stopped more quickly than by oral delivery, it also provides important safety advantages and can allow a patient to continue needed antiplatelet therapy right up until a necessary bleeding event, such as for surgery.

A transdermal patch has several advantages compared to intravenous infusion. A tirofiban patch is easier to administer and can be administered outside of a clinical setting. For example, in an emergency situation (eg. heart attack) attended by first responders, potent intravenous antiplatelet agents, such as tirofiban, cannot be provided due to their complexity and safety concerns associated therewith. The present invention would allow for a relatively simple bolus injection to be given followed by application of a transdermal patch comprising tirofiban. In other instances a transdermal patch, typically developed for use with skin penetration enhancers, can be used to deliver both the initial (eg. bolus) dose and maintain the desired dose thereafter. Transport to a medical facility would then be easier without having an intravenous line and associated infusion pumps for a tirofiban infusion. As another example, use of a transdermal patch would facilitate alternative and more efficient delivery of care to a patient. A patient treated with a transdermal patch would be able to move more rapidly to less intensive treatment wards than if the same patient received the treatment by IV continuous infusion. The patient would also experience less risk of technical or human errors associated with delivery of IV agents. The hospital system would also have several benefits, for example, substantially reduced staff time required to manage administration of the IV infusion.

In an embodiment, a method for inhibiting platelet aggregation comprises 1) administering a bolus injection of tirofiban and 2) administering to the patient, after the bolus injection, 0.1 to 0.15 μg/kg/min of tirofiban for about 12 to about 24 hours, wherein the tirofiban is administered via a transdermal patch. The tirofiban includes salts thereof. In an embodiment the tirofiban could be used as a base or a pharmaceutically acceptable salt (eg tirofiban hydrochloride). A bolus injection of tirofiban can be 15, 20, or 25 μg/kg. In another embodiment, a method for inhibiting platelet aggregation comprises 1) administering 0.4 μg/kg/min of tirofiban to a patient for 30 minutes and 2) administering to the patient, after the intravenous infusion, 0.1 to 0.15 μg/kg/min of tirofiban, wherein the tirofiban is administered via a transdermal patch. Transdermal administration of tirofiban can be administered for about 2 hours to about 5 days. Specifically, the transdermal administration of tirofiban can be administered for 1 hour, 2 hours, 4 hours, 6 hours, 8 hours, 10 hours, 12 hours, 18 hours, 24 hours, 36 hours, 48 hours, 72 hours, 96 hours, or 120 hours, 30 days and greater than 365 days as determined by the physician. The tirofiban includes salts thereof. In an embodiment the tirofiban is tirofiban hydrochloride. In certain embodiments, the transdermal administration can be titrated by the patient.

In another embodiment, a method for inhibiting platelet aggregation comprises administering a total of 15, 20 or 25 μg/kg of tirofiban over a relatively short period of time (commonly referred to as a bolus dose) administered via a transdermal patch. Transdermal administration could be administered from about 1 to 30 minutes, or otherwise as rapidly as the characteristics of the patch permit absorption of the intended dose. Approaches available to an expert in the development of transdermal patch products, such as enhancers and procedures used to increase permeability of the outer skin tissue layer, would be employed to facilitate the more rapid absorption of product required for administration of a bolus dose. The transdermal administration could be developed such that it is able to continue to deliver an effective maintenance dose of the antithromobotic following the bolus dose. Tirofiban could be used as the base or a pharmaceutically acceptable salt.

In another embodiment, a method for inhibiting platelet aggregation comprises administering to a patient a dose of tirofiban selected to provide an intended level of platelet inhibition suitable for the individual patient and for treating his or her specific condition via a transdermal patch. Specifically, transdermal administration of tirofiban can be administered as long as is required to obtain an intended plasma level and resulting platelet inhibition level in order to address the patient's condition or to provide platelet inhibition during the performance of a specific, time limited procedure. The duration of administration may be as little as 15 to 90 minutes, depending on the rate at which the product is absorbed. Tirofiban could be used as the base or a pharmaceutically acceptable salt

An embodiment of the invention includes a transdermal patch comprising tirofiban or salts thereof. A transdermal patch can include one or more backing layers and a matrix layer. A backing layer can include any conventional material

that does not adversely react with any other component of the transdermal patch. A matrix layer can include, but is not limited to, polymeric matrix materials such as polyvinyl alcohols, polyvinyl pyrrolidones, and/or gelatin. A matrix layer can include tirofiban or a salt thereof in solution or suspension.

The tirofiban or salt thereof can be formulated in combination with a carrier or vehicle. A "carrier" or "vehicle" refers to materials without pharmacological activity that are suitable for administration in conjunction with the presently disclosed and claimed compositions, and include any such known carrier materials, e.g., any liquid, gel, solvent, liquid diluent, solubilizer, or the like. Carriers suitable herein are "pharmaceutically acceptable" in that they are nontoxic, do not interfere with drug delivery, and are not for any other reasons biologically or otherwise undesirable. Examples of specific suitable carriers and vehicles for use herein include water, mineral oil, silicone, inorganic gels, aqueous emulsions, liquid sugars, waxes, petroleum jelly, and a variety of other oils and polymeric materials.

In a further embodiment, a transdermal patch can also include a skin penetration enhancer such as N-methyl-2-pyrrolidone, oleic acid, C8-C22 aliphatic alcohol (e.g., oleyl alcohol), sorbitan ester, linoleic acid, or isopropyl linoleate. In addition, a transdermal patch system could include various techniques to improve delivery via addition of chemicals to lasers, micro needles, electrical energy, ultrasound. A transdermal patch of the invention can optionally include a rate controlling membrane.

An embodiment of the patch also includes an adhesive so the patch sticks to a patient without the aid of another product. An adhesive layer of this system can also contain tirofiban or a salt thereof. An adhesive layer containing tirofiban can be a single layer or multilayers. A multilayer adhesive containing tirofiban can be separated by a membrane, including a rate controlling membrane.

Drugs like clopidogrel, aspirin and warfarin are routinely taken by several million people in North America for the prevention of stroke, heart attack and other events related to blood clots. These drugs need to be stopped before a person undergoes surgery or certain procedures because it can cause dangerous amounts of bleeding during and after surgery. However, patients requiring continuous antiplatelet therapy who are scheduled for a surgical intervention, such as CABG or PCI, are at an increased risk of myocardial events during this antithrombotic drug free period. A transdermal tirofiban patch can maintain adequate levels (50-80%) of platelet inhibition in these patients almost right up until the time of the planned surgery. Since tirofiban has a short half -life, it would be eliminated from the body within a few hours after removal of the transdermal patch. In addition, the patient awaiting such a procedure (and on a transdermal patch) does not necessarily have to stay in a hospital in the days leading up to surgery and can arrive at the appropriate surgical facility as little as 1 - 10 hrs before the surgical procedure as deemed necessary by the medical practitioner. Similarly, the patient often requires additional care following the procedure and may be placed under the care of a hospital unit such as a cardiac care unit (CCU) for hours or days following the procedure for observation. Therefore this invention can lead to a better patient care and improved hospital management of CCU facilities.

## Examples

Below are examples of specific embodiments for carrying out the present invention. The examples are offered for illustrative purposes only, and are not intended to limit the scope of the present invention in any way.

Efforts have been made to ensure accuracy with respect to numbers used (e.g., amounts, temperatures, etc.), but some experimental error and deviation should, of course, be allowed for.

## Example 1

Ethylene-vinyl acetate co-polymer (1 Kg, 40 W % vinyl acetate) is weighed into the hopper of a Ross internal mixing bowl (Model PVM-2 or PD-2, Charles Ross & Sons Co., Hauppauge, N.Y.). The bowl is connected to the drive unit of a Brabender Mixing Bowl (Model R.E.O.-6) (C.W. Brabender Instruments, Inc., South Hackensack, N.J.). The top of the bowl is closed and the mixer is operated without heat, until an even melt is obtained from the added pellets (about 0.5 h). At the end of this time the unit is stopped and the lid is opened. Tirofiban (450 gm) is added to the bowl. After the lid is closed, the unit is energized to achieve an even dispersion of tirofiban in the co-polymer (0.5 h). The mixture is removed from the mixer and stored for further use.

A Brabender extrusion machine (0.75 inch diameter, 4 feet, single screw machine, Model 2503) (C.W. Brabender Instruments, Inc., South Hackensack, N.J.), is attached to a similar preparatory drive machine as described above. A heatable four-inch width flex-nip extrusion die is affixed to the end of the extrusion barrel. The extrudate is sandwiched between one interleaving siliconized polyester film and one polyester/EVA backing film. A set of three calender rolls is set up to size the tirofiban-containing layer measuring six inch wide as it exits from the extruder. The target tirofiban reservoir film thickness of 0.1+/-0.01 mm is achieved by appropriate adjustment of the calender rolls. The trilaminate is wound on a take-up roll for further manufacturing use.

A solution acrylate adhesive (product number 87-4287, National Starch and Chemical Corporation, Bridgewater, N.J.; Solutia, Mass.) in ethyl acetate is cast using a casting machine to form an adhesive layer. The solution is pressure-fed from a reservoir pot, through a slot die onto a relatively easy release siliconized polyester film. The film/adhesive bilayer is drawn through the heated stages of a dynamic oven to remove the ethyl acetate to less than 500 μg/gm levels. As the film exits the last stage of the drying ovens, the peelable layer is removed from the tirofiban reservoir film and the adhesive layer is laminated to the available surface of the laminate. The four-layer film (PET/EVA layer, tirofiban reservoir, acrylate adhesive & peelable liner) is wound on take-up rolls for further processing.

Individual transdermal systems are die-cut to 20 $cm^2$ area. In a final manufacturing step, systems are slit and packaged in Surlyn/**Al**/Kraft laminate pouchstock (Alcoa Flexiable Packaging, Richmond, Va.), with a terminal heat-sealing step. The device is capable of delivering tirofiban at 10-50 mg/24 hrs for up to 3 days, preferably for about 12-24 hrs.

## Example 2

The example illustrates the use of a continuous feeder-extruder (such as a Kneader extruder (Model MKS 30) Coperion Corp., Ramsey N.J.). A solid adhesive (such as a melt-processible acrylate, for example SEBS (stryene-ethylene/butylene-stryene) polymers Kraton SEBS G1657, from Kraton Polymers, Houston, Tex.) is continuously fed to a hopper, while tirofiban base is fed into a second hopper on the extruder. The ratio of adhesive to polymer is 4:1. The extruded film is calendered downstream from the extrusion die between a siliconized polyester (3 mil) and a backing film comprised of polyester/polyethylene (2 mil), to a thickness of 0.125, +/-0.0125 mm, at a width of approximately 6.5 inches. The systems are die-cut to an area ranging from about 5 $cm^2$ to about 50 $cm^2$.

To improve transdermal tirofiban flux, the tirofiban reservoir is formulated to contain as much as 15 W % permeation enhancer (for example lauryl proline ester, glycerol monolaurylate or oleic acid). Use of permeation enhancers increases the flux 2-3 times over comparable devices with permeation enhancers.

## Example 3

To improve the tirofiban blood level variation, a rate control membrane can be manufactured and interposed between the tirofiban reservoir and the acrylate layer to regulate the tirofiban release. Depending upon the rate desired, an EVA film of 5-20% vinyl acetate, at a thickness of about 0.05 mm may be inserted.

## Example 4. *In vitro* analysis of tirofiban permeation kinetics.

Permeation of tirofiban from transdermal patches containing different concentrations of permeation enhancers was measured across mouse skin and human skin in Franz diffusion cells (produced by Hanson research).

Two patches having the following formulas are prepared expressed as weight percentages of the single components.

| Constituents | Patch A | Patch B |
|---|---|---|
| Tirofiban | 43 | 45 |
| Duro-Tak® 87-2852 | 53 | 54 |
| Sorbitan oleate | 2 | 0 |
| Propylene glycol | 2 | 1 |

The permeation studies are conducted using Franz diffusion cells modified with an aperture 15 mm in diameter (membrane surface area 1.766cm2) and 7 ml cell volume. A phosphate buffer of pH 7 maintained under constant agitation is used as receptor fluid within the cell.

CDF1 mice aged at a maximum of 6 weeks are killed, and samples of skin taken from their backs are depilated and washed in physiological solution (0.9% NaCl in distilled water).

Within an hour of removal from the animal, the skin samples are placed over the aperture of the cells to form a membrane, and the transdermal patches to be analysed are each placed over a membrane and fastened with clips.

At determined intervals, 300 μl of receptor solution are withdrawn and replaced each time with an equivalent volume of phosphate buffer.

The conditions under which the permeation test is carried out are summarized in the following table:

| | |
|---|---|
| Dimension of patch | 1.766 $cm^2$ |
| Receptor solution | Phosphate buffer at pH 7.4 |

(continued)

| | |
|---|---|
| Volume | 7 ml |
| Temperature | 37° C |
| Times of sample withdrawal | 30 min, 1 hr, 2hr, 4hr, 6hr, 8hr, 24 hrs |

The quantity of tirofiban in each sample is determined by means of HPLC analysis. The results obtained for each patch are analyzed statistically using t-test for independent samples. The results of the t-test indicate that there are no significant differences between skin permeation kinetics obtained with patches A and B. Both patches show a steady and near linear increase in permeation rate over the first 2 hours after application, followed by a near constant permeation rate for between about 2-8 hours, followed, in turn, by a decline in permeation rate. Measurable permeation rates are achieved for over 12 hours

**Example 5. Studies of permeation across human skin:**

Samples of abdominal skin are obtained from the same donor by means of a surgical procedure.
Membranes consisting of the stratum corneum and epidermis are prepared by immersing the skin into distilled water at 60°C ± 1°C for one minute followed by their removal from the dermis. The membranes are later placed in a dryer at about 25% ambient humidity, wrapped in aluminium sheets and maintained at a temperature of about -20°C ± 1°C until required. Dried membrane samples are rehydrated at ambient temperature by immersing in a saline solution for 16 hrs.
Each membrane is mounted onto modified Franz diffusion cells having a receptor volume of 5 ml and diffusion area of 0.636cm$^2$, and fastened by means of clips.
At the start of the experiment, patches having an area of 2.54 cm$^2$ are applied to the diffusion cell as the donor phase.
The receptor liquid is a phosphate buffer at pH 7.4, continuously stirred with a magnetic stirrer and temperature controlled at 37°C ± 1°C, so that the surface of the skin was at a temperature of 32°C ± 1°C. At pre-established intervals (30 min, 1,2,4,6,8 and 24 hrs), 200 μl of the sample are withdrawn from the receptor compartment and replaced with fresh receptor fluid.
The results obtained are analyzed statistically using the t-test for independent samples. No significant differences were seen between skin permeation kinetics obtained with patches A & B. Both patches show a steady and near linear increase in permeation rate over the first 2 hours after application, followed by a near constant permeation rate for between about 2-8 hours, followed, in turn, by a decline in permeation rate. Measurable permeation rates are achieved for over 12 hours.

**Example 6. Phase II/Phase III - Administration of Transdermal and Intravenous Aggrastat in Subjects with Stable Coronary Artery Disease Undergoing Percutaneous Coronary Intervention (PCI)**

A patient is admitted to the hospital to undergo PCI. Two to three hours prior to surgery, the patient is given a initial transdermal dose of tirofiban using the transdermal patch A of Example 1. The patch provides a delivery rate of 0.2 mg/hr or greater, and a total dose of about 2 mg or greater. After approximately one hour a platelet function analysis is done in accordance with the instructions and components of the transdermal titration kit to determine the level of platelet inhibition in the patient. If adequate no additional patches are administered to the patient. If platelet inhibition has not reached the desired percentage then additional transdermal doses are administered to the patient until the desired percent inhibition is achieved.
Following completion of the surgery, the patient is prescribed to take transdermal tirofiban for an extended time as determined by the patient's physician.

**Example 7: Titratable patch**

A series of 7 (seven) patches of varying sizes and dosage strengths are prepared using the general method of Example 1 (patch B). The patches increase in size by increments of 3 cm$^2$, from 3 cm$^2$ to 21 cm$^2$, with each increment corresponding to approximately 100 μg/hr of tirofiban permeation across the skin (ranging to 100 μg/hr to 700 μg/hr).
A 70 year old, 95 kg, diabetic patient is diagnosed with coronary artery disease and is at high risk of cardiac complications and myocardial infarction. The patient is not eligible for surgical or percutaneous intervention to address the disease and requires continual antiplatelet therapy however has also been diagnosed as being unresponsive to available oral therapies. The healthcare professional desires to provide a regimen of chronic antiplatelet therapy that is

carefully controlled to reduce the risk of bleeding and other side effects while achieving the desired target platelet inhibition.

The healthcare professional will first apply the smallest, 3 $cm^2$ patch from the transdermal tirofiban titration kit. This first patch is designed to deliver a dose of 0.1 mg/hr. Based on the time required for the patch to reach its intended dosage rate, approximately 2 hours after the patch is applied, the healthcare professional will then take a small blood sample and utilize the Ultegra / RPFA platelet function analysis system to determine the degree of platelet inhibition achieved by the first patch. Although the degree of platelet inhibition by required varies depending on the indication, in this patient it is 75 - 80%.

The reading from the Ultegra / RPFA shows that the patient's platelets are 53% inhibited. The healthcare professional then refers to the chart provided in the transdermal tirofiban titration kit and, referencing the Ultegra/RPFA result and the age, sex, weight and disease state of the individual patient, and determines that the optimal dose titration strategy is to remove the first 3 $cm^2$ patch and apply a 6 $cm^2$ patch in the same location. This second patch is designed to deliver a dose of 0.2 mg/hr. Approximately 2 hours later, the patient or healthcare professional will again take a small blood sample and utilize the Ultegra / RPFA platelet function analysis system to determine the degree of platelet inhibition achieved. The reading now shows the patient's platelets are 77% inhibited, which is the target inhibition rate for this patient.

Referring again to the chart provided in the transdermal tirofiban titration kit, the patient is prescribed a three month course of therapy for a specific dosage strength of transdermal tirofiban that corresponds with the 6 $cm^2$ patch provided in the kit.

The healthcare professional will be directed to repeat the titration process every three months to ensure the patient is adequately treated for their condition. If based the patient requires adjustment to therapy, the titration process will successively specify higher dosage rate or smaller dosage rate with testing every 2 hours until the desired inhibition level is attained.

**Example 8 - Pre-surgery Treatment**

A 60 year old, 92 kg, patient who has previously had a stent implanted is taking clopidogrel daily for the prevention of stroke, heart attack and other events related to blood clots, and requiring gastric surgery unrelated to their cardiovascular condition, is taken off clopidogrel 7 days before surgery, to prevent dangerous amounts of bleeding during and after the surgery.

In order to mitigate the risk of stroke, heart attack and other events related to blood clots while the patient is not taking clopidogrel, the patient is placed on a regimen of daily transdermal tirofiban patches. At the beginning of the course of therapy, the dosing strength of the patch is titrated (as described generally in Example 4, above) so that the patch maintains 60-80% platelet inhibition in the patient. The patient is then sent home with a 7 day supply of patches corresponding to the suitable dosing strength.

The patch is removed within the prescribed window of 2-8 hours (in this patient it was removed 4 hours prior) before the surgery is scheduled, maximizing the amount of time the patient is provided the benefit of platelet inhibition, while removing most anti-platelet effects prior to the surgery and thereby reducing the risk of dangerous amounts of bleeding during and after the surgery.

**Example-9: A Phase I Pharmacokinetic-Pharmacodynamic Study: Comparing the Clinical Effectiveness of two Transdermal tirofiban preparations in healthy subjects after an intravenous bolus dose of 25μg/kg.**

A Pharmacokinetic-Pharmacodynamic study enrolling forty healthy volunteers is designed to compare the effectiveness of Patch A and Patch B (mentioned earlier) in healthy volunteers. The enrolled subjects are randomized to one of two treatment arms. Both the treatment groups receive an intravenous bolus dose of tirofiban at 25μg/kg, and randomized to receive either Patch A or Patch B.

Blood samples are collected at 0, 15, 30, 45, 60 min, 2 hrs, 4 hrs, 8 hrs, 16 and 24 hrs after application of the transdermal tirofiban patches. Blood for aggregometry is anticoagulated with PPACK 38 μM and the maximum turbidometric *exvivo* aggregation is assessed in platelet rich plasma in response to 20 μM of ADP. In addition the concentration of tirofiban is determined by RIA at the same time periods.

The concentrations of tirofiban after the bolus dose and transdermal application of Patch A or Patch B is similar to each other and inhibit 85-98% platelet aggregation throughout the 24 hour period. The level of platelet inhibition is well correlated between the two patches and is similar to the therapeutic levels reported by Schneider et al, 2003. Earlier Steinhubl et al 2001(GOLD study) had reported that the level of platelet inhibition directly correlates with the risk of myocardial events after a PCI. Based on the above results, both the patches are able to deliver tirofiban required for its therapeutic effects. However the levels of tirofiban attained by Patch A are more consistent as compared to Patch B. There are no significant adverse effects which prevented the application of either Patch A or Patch B of tirofiban.

**Example 10: A Phase I Pharmacokinetic-Pharmacodynamic Study: Comparing the Clinical Effectiveness of two Transdermal tirofiban preparations in healthy subjects after a transdermal bolus dose of 25μg/kg.**

The experiment of Example 6 is repeated, this time using a transdermally-administered bolus dose. The bolus dose is administered at 25 μg/kg, over at most 2 hours. The bolus transdermal dose is followed by transdermal application of either Patch A or Patch B. The results are very similar to what is exhibited utilizing an IV bolus dose, indicating that the bolus dose can be administered using a transdermal patch.

**Example 11: Phase I Pharmacokinetic-Pharmacodynamic Study Comparing the Clinical Effectiveness of two Transdermal tirofiban preparations in healthy subjects**

40 healthy volunteers are randomized to receive Patch A or Patch B. Blood samples are collected and analysed as mentioned in the example above.

Patch A achieves its intended platelet inhibition within 30 minutes - 6 hrs after application, and maintains its antiplatelet effect till 24 hrs of application, whereas Patch B attains its intended platelet inhibition after 2-8 hrs of patch application. The results indicate that Patch A provides a faster onset and consistent level of platelet inhibition as compared to Patch B.

**Example-12: A Phase II Study Comparing the Clinical Effectiveness of Transdermal Tirofiban versus Intravenous Tirofiban in Patients Undergoing Percutaneous Coronary Intervention (PCI).**

A Phase II study enrolling at least two hundred patients scheduled for percutaneous coronary intervention is designed to compare the clinical effectiveness of transdermal tirofiban versus intravenous tirofiban in patients undergoing percutaneous coronary intervention (PCI). The enrolled patients are randomized to one of two treatment arms. Treatment arm-A, receives an intravenous tirofiban bolus (i.e. 25μg/kg) followed by an intravenous tirofiban infusion (i.e. 0.15μg/kg/min). Treatment arm-B receives intravenous tirofiban bolus followed by a transdermal dose of tirofiban as determined in Example 2 according to the amount of platelet inhibition desired (in this case, >90% inhibition of platelet aggregation).

The primary endpoint of the study is cardiac biomarker elevation (Eg. troponin, CKMB) which is not statistically different between the two treatment arms. Secondary endpoints include the incidence of major bleeding and percent platelet aggregation inhibition and, in both cases, are comparable between the two treatment arms. The results indicate that the patch provides similar efficacy with no significant additional risk as compared to intravenous infusion.

## Claims

**1.** A transdermal drug delivery system, comprising:

- a sheet material coated with an adhesive on a first side;
- a pharmaceutical composition contacting a second side of said sheet material and capable of at least partially passively diffusing through said sheet material to said first side; and
- a flexible backing;

wherein:

the flexible backing and the adhesive-coated sheet material form a pocket containing said pharmaceutical composition;
the pharmaceutical composition is incapable of passively diffusing through the flexible backing;
the pharmaceutical composition comprises tirofiban, or a salt or hydrate thereof; and
an adhesive on said adhesive-coated sheet material is capable of adhering to a patient's skin;
further comprising a skin permeation device or a skin penetration enhancer wherein the skin permeation or skin penetration enhancer is selected from the gyoup consisting of N-methyl-2-pyrrolidone, oleic acid, C8-C22 aliphatic alcohol, sorbitan ester, linoleic acid, and isopropyl linoleate.

**2.** A transdermal drug delivery system in the form of a transdermal patch comprising:

- one or more backing layers;
- a matrix layer, wherein the matrix layer comprises a polymeric matrix material selected from the group consisting

of polyvinyl alcohol, polyvinyl pyrrolidone and gelatin, and tirofiban or a salt or hydrate thereof in solution or suspension within said polymeric matrix material.

3. The transdermal drug delivery system of claim 1 or 2, further comprising a system for titration of administration.

4. The transdermal drug delivery system of claim 3 wherein the system for titration of administration comprises a plurality of perforations to facilitate tearing of said drug delivery system into a plurality of sub-patches.

5. A kit comprising (a) an assay selected from the gyoup consisting of a platelet function assay, a platelet reactivity assay, and a receptor occupancy assay, preferably a platelet function assay; (b) a chart or tool for determining titration of administration based on a result of said assay; and (c) and a transdermal delivery system of any one of claims 1-4.

6. Tirofiban for use in a method of providing platelet inhibition, said method comprising:

administering a base dose of tirofiban;
measuring platelet inhibition levels utilizing an assay;
administering an extended duration, adjusted dose of tirofiban based on the results of said assay, wherein the adjusted dose is delivered using the transdermal delivery system as defined in any one of claims 1-4;
optionally, repeating steps (b) and (c) at a regular interval.

7. Tirofiban for use in the method of claim 6 wherein the assay is selected from the group consisting of a platelet function assay, a platelet reactivity assay, and a receptor occupancy assay, preferably a platelet function assay.

8. Tirofiban for use in the method of claim 7 wherein the base dose of tirofiban is administered using a transdermal drug delivery system as defined in claim 1 or 2 or an intravenously administered bolus dose.

9. Tirofiban for use in a method of providing platelet inhibition, said method comprising:

administering a bolus dose of tirofiban in an amount of about 25 ug/kg;
transdermally administering a maintenance dose of tirofiban;.
wherein the maintenance dose is delivered using the transdermal delivery system as defined in any one of claims 1-4;

10. Tirofiban for use in a method of providing platelet inhibition before and/or after a surgery in a patient taking oral and/or non-reversible platelet inhibition medication, said method comprising:

taking the patient off the oral platelet inhibition medication about 2-5 days before and/or after the surgery;
administering a transdermal drug delivery system as defined in any one of claims 1-4 comprising tirofiban and capable of delivering tirofiban to said patient in a quantity such that the patient exhibits a 60-80% platelet inhibition;
removing said transdermal patch 2-8 hours before and/or after the surgery.

11. Transdermal drug delivery system as defined in any one of claims 1-4, comprising tirofiban for use in a method of treating acute coronary syndrome, unstable angina, ST-elevated myocardial infarction, non-ST elevated myocardial infarction, ischemic stroke, post CABG with incomplete revascularization, essential thrombocytosis, deep vein thrombosis, pulmonary embolism, patients allergic and bor with ASA resistance, heparin induced thrombocytopenia, and prior to and during peri-procedural PCI.

## Patentansprüche

1. Transdermales Arzneimittelabgabesystem, umfassend

- ein mit einem Haftmittel auf einer ersten Seite beschichtetes Folienmaterial;
- eine pharmazeutische Zusammensetzung im Kontakt mit einer zweiten Seite des Folienmaterials und das in der Lage ist, zumindest teilweise passiv durch das Folienmaterial zur ersten Seite zu diffundieren; und
- einen flexiblen Träger;

wobei:

der flexible Träger und das haftmittelbeschichtete Folienmaterial eine Tasche bilden, welche die pharmazeutische Zusammensetzung enthält;
die pharmazeutische Zusammensetzung nicht in der Lage ist, passiv durch den flexiblen Träger zu diffundieren;
die pharmazeutische Zusammensetzung Tirofiban oder ein Salz oder Hydrat davon enthält; und
ein Haftmittel auf dem haftmittelbeschichteten Folienmaterial, das in der Lage ist, um an der Haut eines Patienten anzuhaften;
ferner umfassend eine Hautpermeationsvorrichtung oder einen Hautpenetrationsverstärker, wobei der Hautpermeations- oder Hautpenetrationsverstärker ausgewählt ist aus der Gruppe bestehend aus N-Methyl-2-pyrrolidon, Ölsäure, C8-C22 aliphatischem Alkohol, Sorbitanester, Linolsäure und Isopropyllinoleat.

**2.** Transdermales Arzneimittelabgabesystem in Form eines transdermalen Pflasters, umfassend:

- eine oder mehrere Trägerschichten;
- eine Matrixschicht, wobei die Matrixschicht ein polymeres Matrixmaterial umfasst, ausgewählt aus der Gruppe bestehend aus Polyvinylalkohol, Polyvinylpyrrolidon und Gelatine, und Tirofiban oder ein Salz oder Hydrat davon in einer Lösung oder Suspension innerhalb des besagten polymeren Matrixmaterials.

**3.** Transdermales Arzneimittelabgabesystem nach Anspruch 1 oder 2, ferner umfassend ein System für die Titration der Verabreichung.

**4.** Transdermales Arzneimittelabgabesystem nach Anspruch 3, wobei das System für die Titration der Verabreichung eine Vielzahl von Perforationen umfasst, um ein Auseinanderreißen des besagten Arzneimittelabgabesystems in eine Vielzahl von Sub-Pflastern zu erleichtern.

**5.** Kit, umfassend (a) einen Test, ausgewählt aus der Gruppe bestehend aus einem Blutplättchen-Funktionstest, einem Blutplättchen-Reaktivitätstest und einem Rezeptor-Belegungstest, vorzugsweise einem Blutplättchen-Funktionstest; (b) ein Diagramm oder ein Hilfsmittel zur Bestimmung der Titration der Verabreichung basierend auf einem Ergebnis des besagten Tests und (c) ein transdermales Abgabesystem nach einem der Ansprüche 1-4.

**6.** Tirofiban zur Verwendung in einem Verfahren zur Bereitstellung von Blutplättchen-Hemmung, wobei das Verfahren umfasst:

Verabreichen einer Basisdosis von Tirofiban;
Messen des Ausmaßes von Blutplättchen-Hemmung unter Verwendung eines Tests;
Verabreichen einer angepassten Dosis Tirofiban mit verlängerter Dauer, basierend auf den Ergebnissen des Tests, wobei die angepasste Dosis unter Verwendung eines wie in einem der Ansprüche 1-4 definierten transdermalen Abgabesystems abgegeben wird;
gegebenenfalls Wiederholen der Schritte (b) und (c) in einem regelmäßigen Intervall.

**7.** Tirofiban zur Verwendung in dem Verfahren nach Anspruch 6, wobei der Test ausgewählt ist aus der Gruppe bestehend aus einem Blutplättchen-Funktionstest, einem Blutplättchen-Reaktivitätstest und einem Rezeptor-Belegungstest, vorzugsweise einem Blutplättchen-Funktionstest.

**8.** Tirofiban zur Verwendung in dem Verfahren nach Anspruch 7, wobei die Basisdosis von Tirofiban unter Verwendung eines wie in Anspruch 1 oder 2 definierten transdermalen Arzneimittelabgabesystems oder einer intravenös verabreichten Bolusdosis verabreicht wird.

**9.** Tirofiban zur Verwendung in einem Verfahren zur Bereitstellung von Blutplättchen-Hemmung, wobei das Verfahren umfasst:

Verabreichen einer Bolusdosis von Tirofiban in einer Menge von etwa 25 μg/kg;
transdermales Verabreichen einer Aufrechterhaltungsdosis von Tirofiban;
wobei die Aufrechterhaltungsdosis unter Verwendung eines wie in einem der Ansprüche 1-4 definierten transdermalen Arzneimittelabgabesystems abgegeben wird.

**10.** Tirofiban zur Verwendung in einem Verfahren zur Bereitstellung von Blutplättchen-Hemmung vor und/oder nach

einer Operation bei einem Patienten, der orale und/oder nicht-reversible Medikation zur Blutplättchen-Hemmung einnimmt, wobei das Verfahren umfasst:

Absetzen der oralen Medikation zur Blutplättchen-Hemmung beim Patienten ungefähr 2-5 Tage vor und/oder nach der Operation;
Verabreichung eines wie in einem der Ansprüche 1-4 definierten transdermalen Arzneimittelabgabesystems umfassend Tirofiban und das in der Lage ist, an den Patienten Tirofiban in einer solchen Menge abzugeben, dass der Patient eine 60-80 %-ige Blutplättchen-Hemmung aufweist;
Entfernen des transdermalen Pflasters 2-8 Stunden vor und/oder nach der Operation.

**11.** Transdermales Arzneimittelabgabesystem nach einem der Ansprüche 1-4, umfassend Tirofiban zur Verwendung in einem Verfahren zur Behandlung von akutem Koronarsyndrom, instabiler Angina Pectoris, ST-erhöhtem Myokardinfarkt, nicht-ST-erhöhtem Myokardinfarkt, ischämischem Schlaganfall, post-Koronararterien-Bypass (post-CABG) mit unvollständiger Revaskularisierung, essentieller Thrombozytose, tiefe Venenthrombose, Lungenembolie, allergische Patienten und/oder mit ASA-Resistenz, Heparin-induzierte Thrombozytopenie und vor und während peri-prozeduraler perkutane Koronarintervention (PCI).

## Revendications

**1.** Système de délivrance transdermique de médicament, comprenant :

- une feuille de matériau enduite d'un adhésif sur un premier côté ;
- une composition pharmaceutique en contact avec un deuxième côté de ladite feuille de matériau capable de diffuser passivement au moins partiellement à travers ladite feuille de matériau vers ledit premier côté ; et
- un support souple ;

dans lequel :

le support souple et la feuille de matériau enduite d'adhésif forment une poche contenant ladite composition pharmaceutique ;
la composition pharmaceutique est incapable de diffuser passivement à travers le support souple ;
la composition pharmaceutique comprend du tirofiban ou un sel ou un hydrate de celui-ci ; et
l'adhésif sur ladite feuille de matériau enduite d'adhésif est capable d'adhérer à la peau d'un patient ;
comprenant en outre un dispositif de perméation cutanée ou un promoteur de pénétration cutanée, dans lequel le promoteur de perméation cutanée ou de pénétration cutanée est choisi dans le groupe comprenant la N-méthyl-2-pyrrolidone, l'acide oléique, un alcool aliphatique en C8-C22, un ester de sorbitane, l'acide linoléique et le linoléate d'isopropyle.

**2.** Système de délivrance transdermique de médicament sous forme de timbre transdermique comprenant :

- une ou plusieurs couches de support ;
- une couche matricielle, dans lequel la couche matricielle comprend un matériau polymère matriciel choisi dans le groupe comprenant l'alcool polyvinylique, la polyvinyl-pyrrolidone et la gélatine, et le tirofiban ou un sel ou un hydrate de celui-ci en solution ou en suspension dans ledit matériau matriciel polymérique.

**3.** Système de délivrance transdermique de médicament selon la revendication 1 ou 2, comprenant en outre un système de dosage de délivrance.

**4.** Système de délivrance transdermique de médicament selon la revendication 3, dans lequel le système de dosage de délivrance comprend une pluralité de perforations pour faciliter la séparation par déchirure du système de délivrance de médicament en une pluralité de sous-timbres.

**5.** Kit comprenant (a) un test choisi dans le groupe comprenant un test de fonction plaquettaire, un test de réactivité plaquettaire et un test d'occupation de récepteurs, de préférence un test de fonction plaquettaire ; (b) un abaque ou un outil de détermination du dosage de délivrance à partir du résultat dudit test ; et (c) un système de délivrance transdermique selon l'une quelconque des revendications 1 à 4.

**6.** Tirofiban destiné à être utilisé dans un procédé d'inhibition plaquettaire, ledit procédé comprenant :

l'administration d'une dose de base de tirofiban ;
la mesure des niveaux d'inhibition plaquettaire au moyen d'un test ;
l'administration sur une période prolongée d'une dose de tirofiban adaptée à partir des résultats dudit test, dans lequel la dose adaptée est délivrée au moyen du système de délivrance transdermique selon l'une quelconque des revendications 1 à 4 ;
optionnellement, répéter les étapes (b) et (c) à intervalles réguliers.

**7.** Tirofiban destiné à être utilisé dans le procédé selon la revendication 6, dans lequel le test est choisi dans le groupe comprenant un test de fonction plaquettaire, un test de réactivité plaquettaire et un test d'occupation de récepteurs, de préférence un test de fonction plaquettaire.

**8.** Tirofiban destiné à être utilisé dans le procédé selon la revendication 7, dans lequel la dose de base de tirofiban est administrée au moyen d'un système de délivrance transdermique de médicament selon la revendication 1 ou 2 ou d'une dose bolus administrée par intraveineuse.

**9.** Tirofiban destiné à être utilisé dans un procédé d'inhibition plaquettaire, ledit procédé comprenant :

l'administration d'une dose bolus de tirofiban d'une quantité de 25 ug/kg environ ;
l'administration transdermique d'une dose d'entretien de tirofiban ;
dans lequel la dose d'entretien est délivrée au moyen du système de délivrance transdermique selon l'une quelconque des revendications 1 à 4.

**10.** Tirofiban destiné à être utilisé dans un procédé d'inhibition plaquettaire avant et/ou après une intervention chirurgicale chez un patient prenant une médication d'inhibition plaquettaire orale et/ou non réversible, ledit procédé comprenant :

l'arrêt de la médication orale d'inhibition plaquettaire environ 2 à 5 jours avant et/ou après l'intervention chirurgicale ;
l'administration d'un système de délivrance transdermique de médicament selon l'une quelconque des revendications 1 à 4 comprenant du tirofiban et capable de délivrer du tirofiban audit patient en une quantité telle que le patient présente une inhibition plaquettaire de 60 à 80 % ;
le retrait du timbre transdermique de 2 à 8 heures avant et/ou après l'intervention chirurgicale.

**11.** Système de délivrance transdermique de médicament selon l'une quelconque des revendications 1 à 4, comprenant du tirofiban destiné à être utilisé dans un procédé de traitement du syndrome coronarien aigu, de l'angine instable, de l'infarctus du myocarde avec surélévation du segment ST, de l'infarctus du myocarde sans surélévation du segment ST, de l'accident vasculaire ischémique, d'une revascularisation incomplète après pontage aorto-coronarien, de la thrombocytose essentielle, de la thrombose veineuse profonde, de l'embolie pulmonaire, chez les patients allergiques et résistants à VAAS, de la thrombocytopénie induite par l'héparine et avant et pendant une ICP péri procédurale.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 61161132 B
- US 61240021 B
- US 61259683 B
- WO 0203969 A
- US 2003207918 A
- WO 2004036226 A
- US 6770660 B
- US 5292756 A